# EUROPEAN PATENT SPECIFICATION

(11) **EP 1 232 141 B1**
(45) Date of publication and mention of the grant of the patent: **06.10.2004**
(21) Application number: 00980588.8
(22) Date of filing: 21.11.2000
(51) Int. Cl.: C07C 217/74, C07C 213/06, A61K 31/135, A61P 25/24

(54) **ETHERS OF O-DESMETHYL VENLAFAXINE**
O-DESMETHYLVENLAFAXIN-ETHER
ETHERS DE O-DESMETHYL VENLAFAXINE

(30) Priority: 24.11.1999 US 448268
(43) Date of publication of application: 21.08.2002
(73) Proprietor: Wyeth, Madison, New Jersey 07940 (US)
(72) Inventor: YARDLEY, John, Patrick, King of Prussia, PA 19406 (US); ABOU-GHARBIA, Magid, Abdel-Megid, Princeton, NJ 08550 (US); ULLRICH, John, William, Exton, PA 19341 (US)
(74) Representative: Connelly, Michael John
(86) International application number: PCT/US2000/031895
(87) International publication number: WO 2001/038293

(56) References cited:
- YARDLEY J P ET AL: "2-PHENYL-2-(1-HYDROXYCYCLOALKYL)ETHYLAMIN E DERIVATIVES: SYNTHESIS AND ANTIDEPRESSANT ACTIVITY" JOURNAL OF MEDICINAL CHEMISTRY,AMERICAN CHEMICAL SOCIETY. WASHINGTON,US, vol. 33, 1990, pages 2899-2905, XP000891765 ISSN: 0022-2623 cited in the application

## Description

This invention relates to ethers of O-desmethyl venlafaxine, more particularly to O-α-acyloxyalkyl ethers of 4-[2-(Dimethylamino-1-(1-hydroxycyclohexyl)ethyl]-phenol, processes for preparing them as well as pharmaceutical compositions and uses thereof.

### Background of the Invention

Various patents and literature references describe the biological activities of venlafaxine, and its salts and analogs. Venlafaxine hydrochloride tablets are marketed by Wyeth-Ayerst Laboratories as EFFEXOR.

The absolute configuration of the (+) enantiomer of venlafaxine was established as S by a single crystal X-ray analysis of the hydrobromide salt and the anomalous dispersion technique (Yardley et al., J. Med. Chem., 1990, 33, 2899).

(R/S)-1-[2-(dimethylamino)-1-(4-methoxyphenyl)ethyl]cyclohexanol and its metabolites 1-[2-(dimethylamino)-1-(4-hydroxyphenyl)ethyl]cyclohexanol and 1-[1-(4-methoxyphenyl)-2-(methylamino)ethyl]cyclohexanol are disclosed and claimed in U.S. Patent No. 4,535,186 (Husbands et al.). U.S. Patent No. 5,530,013 (Husbands et al.) claims the use of venlafaxine in the inducement of cognition enhancement. U.S. Patent No. 5,506,270 (Upton et al.) claims venlafaxine's use in methods of treating hypothalamic amenorrhea in non-depressed women.

U.S. Patents Nos. 5,788,986 (Dodman) and 5,554,383 (Dodman) teaches and claims the use of serotonin reuptake inhibitors in modifying the behavior of dogs.

### Detailed Description of the Invention

This invention provides pharmaceutically active O-α-acyloxyalkyl ethers of the venlafaxine metabolite 4-[2-(Dimethylamino-1-(1-hydroxycyclohexyl)ethyl] phenol ("O-Desmethyl venlafaxine" or "ODV") having the structural formula I 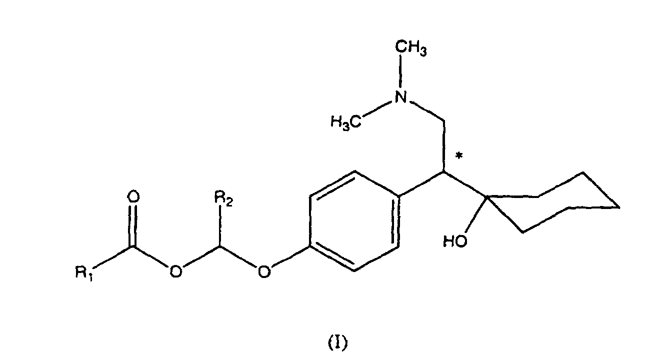 wherein
the configuration at the steriogenic center (*) may be R, S, or RS (the racemate);
R₁ is selected from C₁ - C₆ alkyl, C₁ - C₆ alkoxy, C₃ - C₆ cycloalkyl, or the moiety: 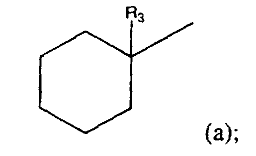 R₂ is selected from H, or C₁-C₆ alkyl; or,
R₁ and R₂ may be concatenated such that 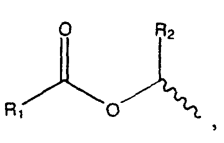 form a moiety having formula (b): 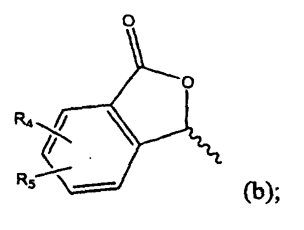 R₃ is selected from H or C₁ - C₆ alkyl; and
R₄ and R₅ are independently selected from H, C₁ - C₆ alkyl, C₃-C₆ cycloalkyl, C₁ - C₆ alkoxy, C₁ - C₆ thioalkoxy, -CN, -OH, -CF₃, -OCF₃, halogen, -NH₂, -NO₂, or mono or dialkylamino wherein each alkyl group has 1 to 6 carbon atoms, for example, -N(CH₃)₂, or pharmaceutically acceptable salts or hydrates thereof.

In some preferred embodiments of the present invention R₁ is t-butyl, methoxy, or isobenzofuranone.

In other preferred embodiments of the invention R₂ is C₁ - C₃ alkyl and in still more preferred embodiments of the invention R₂ is methyl.

Specific examples of compounds of Formula I include:
{4-[2-(dimethylamino)-1-(1-hydroxycyclohexyl)ethyl]phenoxy}methyl pivalate;
1-{4-[2-(dimethylamino)-1-(1-hydroxycyclohexyl)ethyl]phenoxy}ethyl propionate; and
3- {4-[2-(dimethylamino)-1-(1-hydroxycyclohexyl)ethyl]phenoxy}-2-benzofuran-1(3H)-one.

Particularly, this invention provides compounds and/or compositions of both the O-α-acyloxyalkyl R-ether of Formula I and the O-α-acyloxyalkyl S-ether of Formula I, both being substantially free of the other. In addition, the invention provides the O-α-acyloxyalkyl RS-ether of 4-[2-(Dimethylamino)-1-(1-hydroxycyclohexyl)ethyl]-phenol of Formula I.

Substantially free, as used herein means the compound or composition is made up of significantly greater proportion of the desired isomer than of the optical antipode. In a preferred embodiment of the invention, "substantially free" means that the compound or composition is made up of at least about 90% of the desired isomer and about 10% or less of the optical antipode. In still more preferred embodiments of the present invention, the compound or composition is made up of at least about 95% of the desired isomer and about 5% or less of the optical antipode. In yet further embodiments of the present invention the compound or composition is made up of at least about 99% of the desired isomer and about 1% or less of the optical antipode. Preferably the characterized or separated enantiomer will exhibit physical properties of a fully characterized compound, i.e. a uniform melting point and a uniform rotation of plane-polarized light in a polarimeter. Most preferably, the enantiomers will be recrystallized to analytical purity.

C₁ - C₆ alkyl as used herein, such as in the definition of R₁, includes straight or branched chain alkyl groups within the specified range of carbon atoms, eg methyl, ethyl, propyl, n-butyl or t-butyl.

Halogen, as used herein refers to chlorine, bromine, iodine and fluorine.

Pharmaceutically acceptable salts refer to salts prepared from pharmaceutically acceptable acids, including inorganic acids and organic acids, such as, but not limited to, acetic, benzenesulfonic, benzoic, camphorsulfonic, citric, ethenesulfonic, fumaric, gluconic, glutamic, hydrobromic, hydrochloric, isethionic, lactic, maleic, malic, mandelic, methanesulfonic, mucic, mitric, pamoic, pantothenic, phosphoric, succinic, sulfuric, tartaric, p-toluenesulfonic and the like.

Compounds of the invention are readily prepared by methods known in the art, for instance, as described by Bodor, et al., *J. Org. Chem*.(48) 5280-5284 (1983). Where necessary any reactive substituent group or atom may be protected prior to any reaction and deprotected afterwards.

Accordingly this invention provides a process for preparing a compound of formula (I) as defined herein, or a pharmaceutically acceptable salt or hydrate thereof, which process comprises one of the following:
(i) reacting R-, S-, or (R/S)- 4-[2-(dimethylamino-1-(1-hydroxycyclohexyl)ethyl]-phenol of formula: 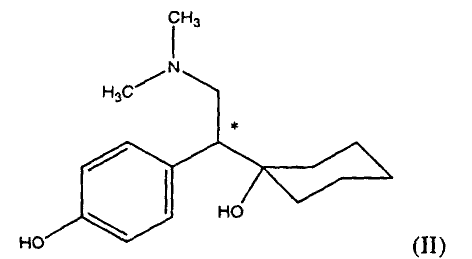 or a salt thereof, with a compound having the formula (V)

   R₁-CO-CHR₂-X (V)

   where R₁ and R₂ are as defined above subject to the proviso that a reactive substituent group, eg an -OH or -NH₂ substituent on the concatenated R₁ and R₂ group may be protected by a protecting group that is subsequently removed and X is a leaving group for example a halogen atom such as chlorine, bromine or preferably iodine; or
(ii) subjecting a compound having formula (IV) 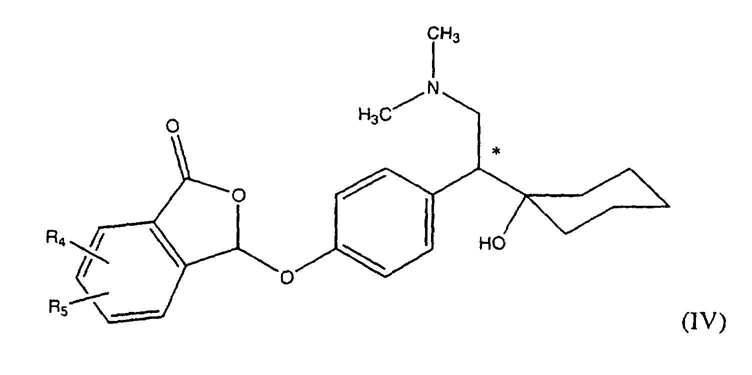 wherein:
   the configuration at the stereogenic center (*) may be R, S, or RS (the racemate) and R₄ and R₅ are independently selected from H, C₁ - C₆ alkyl, C₁ - C₆ alkoxy, C₁ - C₆ thioalkoxy, -CN, -OH, -CF₃, -OCF₃, halogen, -NH₂, -NO₂, or -N(CH₃)₂ subject to the proviso that at least one of R₄ and R₅ is -NO₂, or a pharmaceutically acceptable salt or salt hydrate of such a compound, to reduction to give a compound having formula (IV) wherein R₄ and R₅ are as defined above subject to the proviso that at least one of R₄ and R₅ is -NH₂, or a pharmaceutically acceptable salt or salt hydrate of such a compound;
      or
   (iii) separating a compound having formula (I) wherein R₁ and R₂ are as defined under formula (I) in the form of an enantiomeric mixture so as to isolate a particular enantiomeric form;
      or
   (iv) converting a compound having formula (I) wherein R₁ and R₂ are as defined under formula (I) into a pharmaceutically acceptable salt or salt hydrate thereof by addition of an acid.

With regard to process (i) above the appropriate R-, S-, or (R/S)- 4-[2-(Dimethylamino-1-(1-hydroxy-cyclohexyl)ethyl]phenol is reacted with the appropriate O-α-acyloxyalkyl halide (examples: pivaloyloxymethyl chloride, 3-bromophthalide, iodomethyl pivalate) (Scheme Ia) or (acyloxy)benzyl α-halide (Scheme Ib) in an inert solvent (acetonitrile, tetrahydrofuran, dimethylformamide) in the presence of an alkali metal carbonate (sodium or potassium carbonate) or transition metal carbonate (silver carbonate) in accordance with Schemes Ia and Ib. 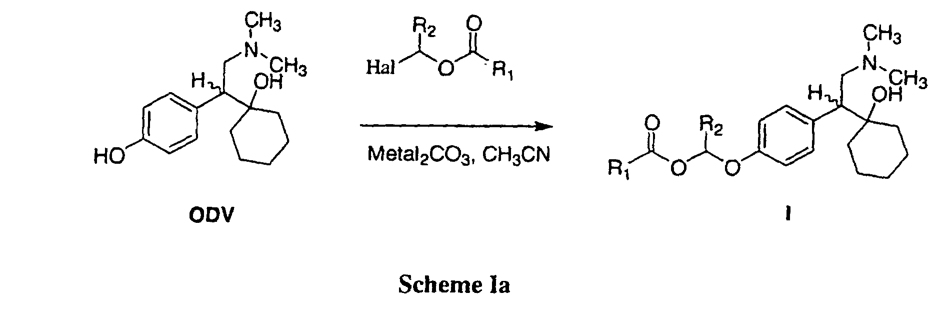 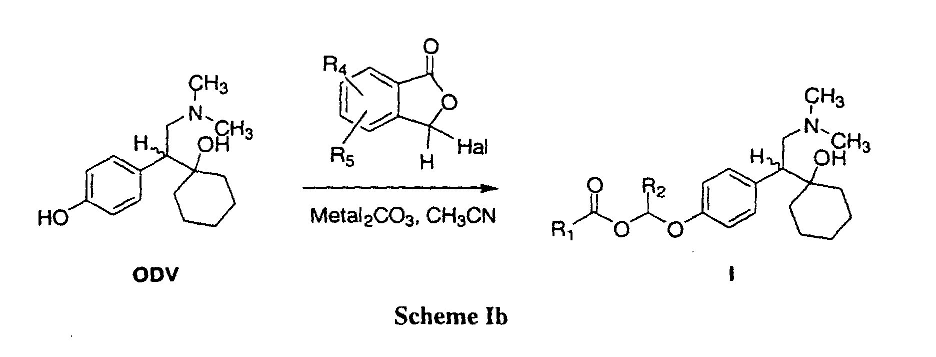 wherein R₁ is selected from C₁ - C₆ alkyl, C₁ - C₆ alkoxy, C₃ - C₆ cycloalkyl, or the moiety: 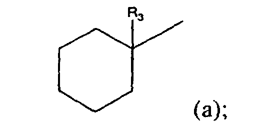 R₂ is selected from H, or C₁- C₆ alkyl;
or R² and R³ are concatenated to form a moiety having formula (b);
R³ is selected from H or C₁ - C₆ alkyl; and
R₄ and R₅ are independently selected from H, C₁ - C₆ alkyl, C₃ - C₆ cycloalkyl, C₁ - C₆ alkoxy, C₁ - C₆ thioalkoxy, -CN, -OH, -CF₃, -OCF₃, halogen, -NH₂, -NO₂, or mono or dialkylamino wherein each alkyl group has 1 to 6 carbon atoms.

In some preferred embodiments of the invention increased yield may be obtained by reacting the appropriate R-, S-, or (R/S)- 4-[2-(Dimethylamino-1-(1-hydroxycyclohexyl)ethyl]phenol with the appropriate O-α-acyloxyalkyliodide in an inert solvent (acetonitrile, tetrahydrofuran, dimethylformamide) in the presence of alkali metal carbonate such as potassium carbonate, or transition metal carbonate such as silver carbonate. Most preferred is the use of O-α-acyloxyalkyliodide in the presence of silver carbonate at low temperatures in the range of approximately 0-5° C.

In a minor modification, compounds of formula I wherein R₁ and R₂ are concatenated to form 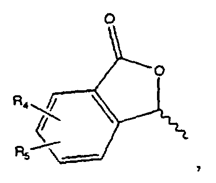 and one or both of R₄ and R₅ are NH₂, can be obtained by catalytic reductions, such as with palladium catalysts, from corresponding analogs wherein R₄ or R₅ are NO₂.

Racemic 1-[2-(dimethylamino)-1-(4-hydroxyphenyl)ethyl]cyclohexanol can be produced as described in Example 26 of U.S. Patent No. 4,535,186 (Husbands et al.), which is incorporated herein by reference. It will be understood that the enantiomers may be separated from each other by standard resolution techniques known in the art.

Alternatively, these R and S enantiomers may be obtained by O-demethylation of the separated enantiomers of venlafaxine using either boron tribromide or ethane thiol anion.

O-α-acyloxyalkyl ethers of Formula I and their pharmaceutically useful salts and hydrates are useful for the biological and pharmacological activities for which venlafaxine and its salts are known in the art. These O-α-acyloxyalkyl ethers may be used in treating or inhibiting central nervous system disorders, including depression, (including but not limited to major depressive disorder, biopolar disorder, and dysthymia), fibromyalgia, anxiety, panic disorder, agorophobia, post-traumatic stress disorder, premenstrual dysphoric disorder (also known as pre-menstrual syndrome), attention deficit disorder (with and without hyperactivity), obsessive compulsive disorder (including trichotillomania), social anxiety disorder, generalized anxiety disorder, autism, schizophrenia, obesity, anorexia nervosa, bulimia nervosa, Gilles de la Tourette Syndrome, vasomotor flushing, cocaine and alcohol addiction, sexual dysfunction (including premature ejaculation), borderline personality disorder, chronic fatigue syndrome, urinary incontinence, pain (including but not limited to migraine, chronic back pain, phantom limb pain, central pain, neuropathic pain such as diabetic neuropathy and postherpetic neuropathy), Raynaud's syndrome, and others. These compounds are also useful in the inducement of cognition enhancement and in regimens for cessation of smoking or other tobacco uses.

This invention also includes methods of treating, preventing, inhibiting or alleviating each of the maladies listed above in a mammal, preferably in a human, the methods comprising providing a pharmaceutically effective amount of a compound of this invention to the mammal in need thereof.

"Providing" as used herein with respect to providing a compound or substance covered by the invention, means either directly administering such a compound or substance, or administering a prodrug, derivative or analog which forms an equivalent amount of the compound or substance within the body.

A pharmaceutically effective dose will include those doses which provide the relief or prevention sought for the malady in question. The compounds of this invention may be provided in the dosages and pharmaceutical formulations known in the art as useful for venlafaxine hydrochloride (such as those doses known for the venlafaxine hydrochloride products marketed by Wyeth-Ayerst Laboratories under the Effexor® trademark). It will be understood that the initial dose, increases therefrom and final daily administration will be determined by a medical professional considering the needs and conditions for each recipient. For instance, a daily dose for an adult human may be from about 75 mg to about 450 mg per day, preferably between about 75 and about 225 mg per day. An initial dose of 75 mg per day may be administered, with increases as determined by a medical professional.

This invention also includes pharmaceutical compositions comprising a pharmaceutically effective amount of a compound of this invention and one or more pharmaceutically acceptable carriers or excipients. A preferred method of administration includes the use of the present compounds in extended release formulations of the type described in published PCT application WO 99/22724 (Sherman et al.), which is incorporated herein by reference.

The present invention is exemplified, but not limited by, the following specific examples.

### Example 1

### {4-[2-(Dimethylamino)-1-(1-hydroxy cyclohexyl)ethyl]phenoxy} methyl pivalate.

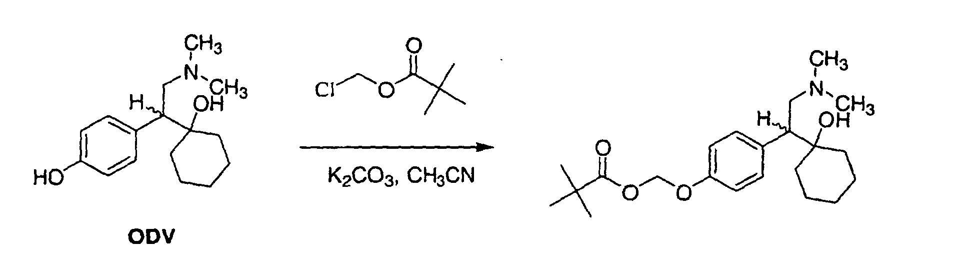

4-[2-(Dimethylamino)-1-(1-hydroxycyclohexyl) ethyl]phenol (1g, 3.79mmol), chloromethyl pivalate (0.75g, 5mmol), anhydrous K₂CO₃ (0.7 g, 5 mmol) and KI (75mg, 0.5 mmol) were stirred in acetonitrile (50mL) and refluxed overnight. The solvent was evaporated and the residue was partitioned between ethyl acetate and water. The ethyl acetate was dried (MgSO₄) and evaporated to give the title compound as a minor component. IR (KBr) 1758cm⁻¹.
MS(+)FAB[M+H]⁺378.3 calcd. For C₂₂H₃₅NO₄ 377.

### Example 2

### {4-[2-(dimethylamino)-1-(1-hydroxycyclohexyl)ethyl]phenoxy}methyl pivalate

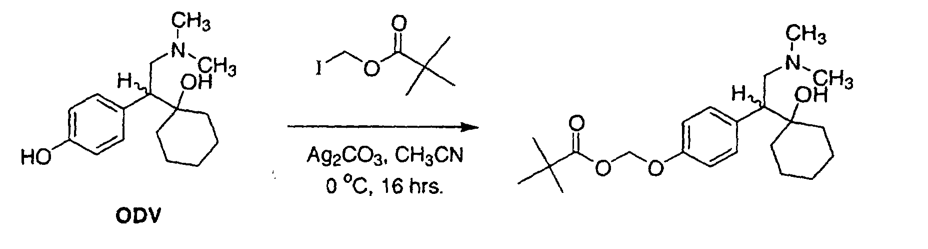

To a solution of ODV (2.0 g, 7.6 mmol) and silver carbonate (8.4 g, 30.4 mmol) in acetonitrile (60 mL) at 0 "C was added a solution of iodomethyl pivalate (prepared as described by Bodor, et al., *J. Org. Chem*. 1983, 48, 5280-5284.) (3.4 g, 14.0 mmol) in acetonitrile (100 mL) dropwise over 4 hr. The reaction mixture was filtered through diatomaceous earth (CELITE, Celite Corporation, Lompoc, CA), then absorbed onto a activated magnesium silicate (60-100 mesh) (FLORISIL, U.S. Silica Company). and purified by column chromatography (FLORISIL, ethyl acetate: acetonitrile 9:1) to afford the title compound (0.87 g, 45 %, based on 68 % conversion) as a yellow tinted semi-solid: ¹H NMR (CD₃CN) δ 0.78-1.0 (m, 2H), 1.19 (s, 9H), 1.15-1.35 (m, 4H), 1.4-1.7 (m, 4H), 2.2 (s, 6H), 2.25 (dd, J = 11.2, 4.5 Hz, 1H), 2.94 (dd, J = 11.2, 4.5 Hz, 1H), 3.22 (t, *J* = 11.2 Hz, 1H), 5.7 (s, 2H), 6.95 (d, *J* = 8.7 Hz, 1H), 7.13 (d, *J* = 8.7 Hz, 1H); ¹³C-NMR (CD₃CN) δ 22.22, 22.44, 26.91, 27.10 (t), 32.83, 38.78 (t), 39.55 (s), 45.71 (q), 52.58, 61.74, 74.45 (d), 86.78 (t), 116.54, 131.48 (d), 136.68, 156.44, 178.05 (s); MS (ESI) *m*/*z* 378 (M+H)⁺; further characterized as the maleate salt. Anal. (C₂₆H₃₉NO₈-0.25H₂O) Calc: C: 62.69, H: 7.99, N: 2.81, Found: C: 62.68, H: 7.68, N: 2.65.
The celite cake was taken up in brine and extracted with ethyl acetate. Evaporation of the solvent affords 0.65 g (33%) recovered ODV.

### Example 3

### 4-[(1R)-2-(dimethylamino)-1-(1-hydroxycyclohexyl)ethyl]phenyl pivalate

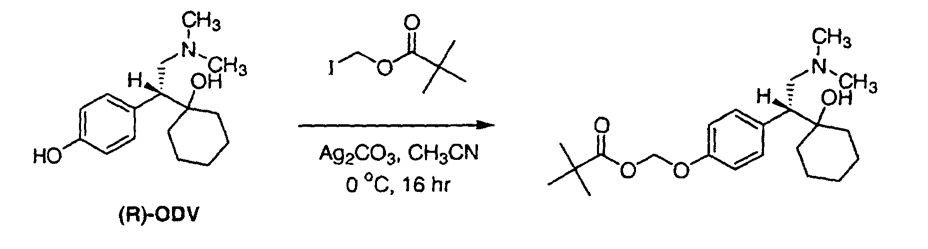

To a solution of ODV (3.0 g, 11.4 mmol) and silver carbonate (12.6 g, 45.6 mmol) in acetonitrile (300 mL) at 0 "C was added a solution of iodomethyl pivalate (prepared as described by Bodor, et al., *J. Org. Chem.* 1983, 48, 5280-5284.) (6.9 g, 28.5 mmol) in acetonitrile (40 mL) in eight equal portions over 16 hr. The reaction mixture was filtered through diatomaceous earth (CELITE, Celite Corporation, Lompoc, CA), then absorbed onto activated magnesium silicate (60-100 mesh) (FLORISIL, U.S. Silica Company) and purified by column chromatography (FLORISIL, ethyl acetate: acetonitrile 9:1) to afford the title compound (1.15 g, 39 %, based on 60 % conversion) as a white foam: ¹H NMR (CD₃CN) δ 0.78-1.0 (m, 2H), 1.19 (s, 9H), 1.15-1.35 (m, 4H), 1.4-1.7 (m, 4H), 2.2 (s, 6H), 2.25 (dd, J = 11.2, 4.5 Hz, 1H), 2.94 (dd, J = 11.2, 4.5 Hz, 1H), 3.22 (t, *J* = 11.2 Hz, 1H), 5.7 (s, 2H), 6.95 (d, *J* = 8.7 Hz, 1H), 7.13 (d, *J* = 8.7 Hz, 1H); ¹³C-NMR (CD₃CN) δ 22.22, 22.44, 26.91, 27.10 (t), 32.83, 38.78 (t), 39.55 (s), 45.71 (q), 52.58, 61.74, 74.45 (d), 86.78 (t), 116.54, 131.48 (d), 136.68, 156.44, 178.05 (s); [α]²⁰_{D} -5.95° (*c* 1.00, MeOH); MS (ESI) *m*/*z* 378 (M+H)+.
The CELITE cake was taken up in brine and extracted with ethyl acetate. Evaporation of the solvent affords 1.2 g (40%) recovered ODV.

### Example 4

### 4-[(1S)-2-(dimethylamino)-1-(1-hydroxycyclohexyl)ethyl]phenyl pivalate

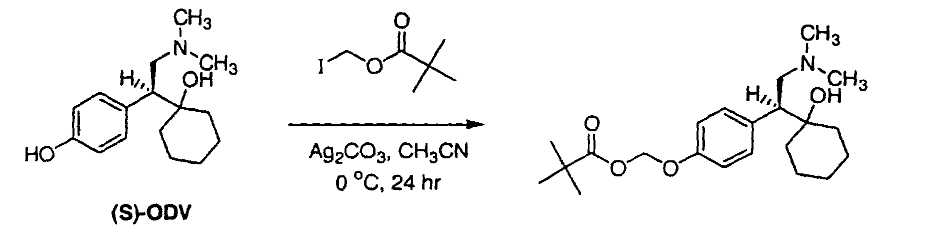

To a solution of ODV (4.0 g, 15.2 mmol) and silver carbonate (16.8 g, 60.8 mmol) in acetonitrile (400 mL) at 0 °C was added a solution of iodomethyl pivalate (prepared as described by Bodor, et al., *J. Org. Chem*. 1983, 48, 5280-5284.) (8.3 g, 34.3 mmol) in acetonitrile (150 mL) over a 9 hr period. The reaction mixture was filtered through diatomaceous earth (CELITE, Celite Corporation, Lompoc, CA), then absorbed onto activated magnesium silicate (60-100 mesh) (FLORISIL, U.S. Silica Company). and purified by column chromatography (FLORISIL, ethyl acetate: acetonitrile 9:1) to afford the title compound (1.58 g, 48 %, based on 57 % conversion) as a clear viscous oil: ¹H NMR (CD₃CN) δ 0.78-1.0 (m, 2H), 1.19 (s, 9H), 1.15-1.35 (m, 4H), 1.4-1.7 (m, 4H), 2.2 (s, 6H), 2.25 (dd, J = 11.2, 4.5 Hz, 1H), 2.94 (dd, J = 11.2, 4.5 Hz, 1H), 3.22 (t, *J* = 11.2 Hz, 1H), 5.7 (s, 2H), 6.95 (d, *J* = 8.7 Hz, 1H), 7.13 (d, *J* = 8.7 Hz, 1H); ¹³C-NMR (CD₃CN) δ 22.22, 22.44, 26.91, 27.10 (t), 32.83, 38.78 (t), 39.55 (s), 45.71 (q), 52.58, 61.74, 74.45 (d), 86.78 (t), 116.54, 131.48 (d), 136.68, 156.44, 178.05 (s); [α]²⁰_{D} +7.23° (*c* 1.00, MeOH); MS (ESI) *m*/*z* 378 (M+H)+.
The CELITE cake was taken up in brine and extracted with ethyl acetate. Evaporation of the solvent affords 1.7 g (43%) recovered ODV.

### Example 5

### {4-[2-(dimethylamino)-1-(1-hydroxycyclohexyl)ethyl]phenoxy}methyl pivalate Maleate salt

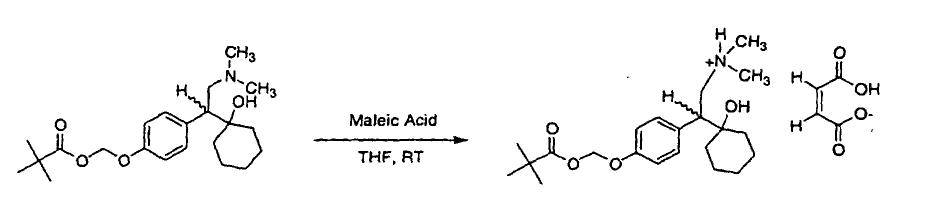

To a solution of {4-[2-(dimethylamino)-1-(1-hydroxycyclohexyl)ethyl]phenoxy}-methyl pivalate (0.032 g, 0.085 mmol) prepared as described in Example 1, in THF (1.0 mL) at RT was added a solution of maleic acid (0.007 g, 0.06 mmol) in THF (1.0 mL). The mixture was warmed and diluted with hexane. The solution was cooled and the resulting crystals filtered off giving the desired maleate salt as a white solid: mp 112-113 °C, ¹H NMR (DMSO-*d*₆) δ 0.9-1.6 (m, 10H), 1.13 (s, 9H), 2.7 (br.s, 6H), 2.97 (m, 1H), 3.55 (m, 2H), 4.59 (br.s, 1H), 5.78 (s, 2H), 6.02 (s, 2H), 7.03 (d, *J* = 8.6 Hz, 1H), 7.33 (d, *J* = 8.6 Hz, 1H), 8.4 (br.s, 1H); MS (ESI) *m*/*z* 378 (M+H)+; (C₂₆H₃₉NO₈-0.25H₂O) Calc: C: 62.69, H: 7.99, N: 2.81, Found: C: 62.68, H: 7.68, N: 2.65.

## Claims

1. A compound of the Formula (I): 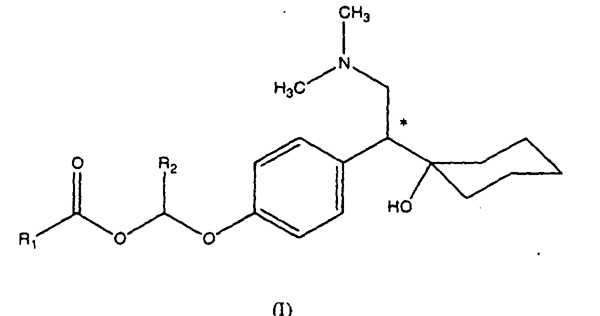 wherein
the configuration at the steriogenic center (*) may be R, S, or RS (the racemate);
R₁ is selected from C₁ - C₆ alkyl, C₁ - C₆ alkoxy, C₃ - C₆ cycloalkyl, or the moiety: 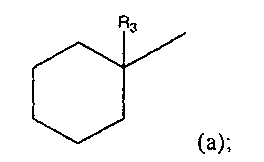 R₂ is selected from H, or C₁- C₆ alkyl; or,
R₁ and R₂ may be concatenated such that 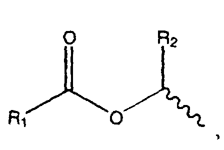 form a moiety having formula (b): 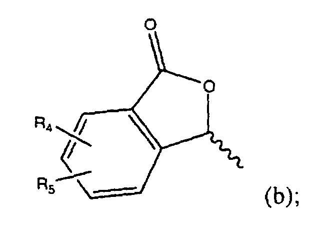 R₃ is selected from H or C₁ - C₆ alkyl; and
R₄ and R₅ are independently selected from H, C₁ - C₆ alkyl, C₃ - C₆ cycloalkyl, C₁ - C₆ alkoxy, C₁ - C₆ thioalkoxy, -CN, -OH, -CF₃, -OCF₃, halogen, -NH₂, -NO₂, or mono or dialkylamino wherein each alkyl group has 1 to 6 carbon atoms, or a pharmaceutically acceptable salt or hydrate thereof.

2. A compound of Claim 1 wherein R₁ is C₁ - C₆ alkyl or C₁ - C₆ alkoxy.

3. A compound of Claim 1 or Claim 2 wherein R₂ is C₁ - C₆ alkyl.

4. A compound of Claim 1 wherein R₁ and R₂ are concatenated such that 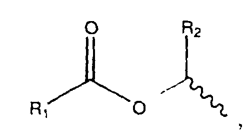 form a moiety having formula (b): 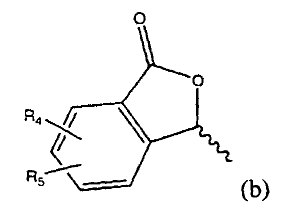 and R₄ and R₅ are hydrogen.

5. A compound of Claim 1 which is {4-[2-(Dimethylamino)-1-(1-hydroxy cyclohexyl)ethyl]phenoxy} methyl pivalate, or a pharmaceutically acceptable salt or hydrate thereof.

6. A compound of Claim 1 which is 1-{4-[2-(dimethylamino)-1-(1-hydroxycyclohexyl)ethyl]phenoxy}ethyl propionate, or a pharmaceutically acceptable salt or hydrate thereof.

7. A compound of Claim 1 which is 3-{4-[2-(dimethylamino)-1-(1-hydroxycyclohexyl)ethyl]phenoxy}-2-benzofuran-1(3H)-one, or a pharmaceutically acceptable salt or hydrate thereof.

8. A pharmaceutical composition comprising a compound of Formula I as claimed in any one of claims 1 to 7, or a pharmaceutically acceptable salt or hydrate thereof; and a pharmaceutically acceptable carrier or excipient.

9. Use of a compound for making a medicament for treating disorders of the central nervous system in a mammal, wherein the compound used is a compound of Formula I as claimed in any one of claims 1 to 7, or a pharmaceutically acceptable salt or hydrate thereof.

10. The use of Claim 9 wherein the central nervous system disorder is selected from one or more of the following:
depression; generalized anxiety disorder; panic disorder; post traumatic stress disorder; attention deficit disorder, with and without hyperactivity; anxiety; schizophrenia; cocaine addiction; alcohol addiction; premenstrual dysphoric disorder and autism.

11. The use of Claim 9 wherein the central nervous system disorder is anorexia nervosa, bulimia nervosa, vasomotor flushing, and chronic fatigue syndrome.

12. The use of Claim 9 wherein the central nervous system disorder is urinary incontinence.

13. The use of Claim 9 wherein the central nervous system disorder is pain.

14. The use of Claim 9 wherein the central nervous system disorder is sexual dysfunction.

15. Use of a compound for making a medicament for enhancing cognition in a mammal, wherein the compound used is a compound of Formula I as claimed in any one of claims 1 to 7, or a pharmaceutically acceptable salt or hydrate thereof.

16. A process for the preparation of a compound having the formula I as claimed in claim 1, or a pharmaceutically acceptable salt or hydrate thereof, which process comprises one of the following:
(i) reacting R-, S-, or (R/S)- 4-[2-(dimethylamino-1-(1-hydroxycyclohexyl)-ethyl]-phenol of formula: 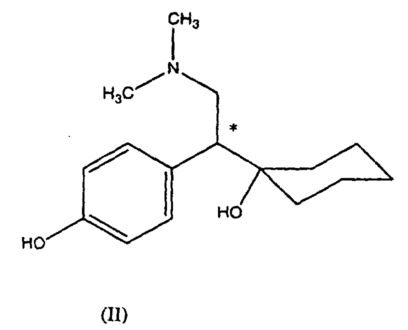 with a compound having the formula (V)
R₁-CO-CHR₂-X (V)
where R₁ and R₂ are as defined above subject to the proviso that an -OH or -NH₂ substituent on the concatenated R₁ and R₂ group may be protected by a protecting group that is subsequently removed and X is a leaving group; or
(ii) reducing a compound having formula (IV) 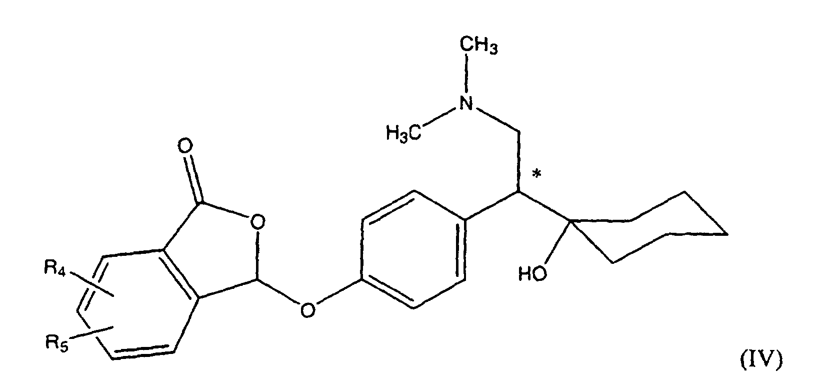 wherein the configuration at the stereogenic center (*) may be R, S, or RS (the racemate) and R₄ and R₅ are independently selected from H, C₁ - C₆ alkyl, C₁ - C₆ alkoxy, C₁ - C₆ thioalkoxy, -CN, -OH, -CF₃, -OCF₃, halogen, -NH₂, -NO₂, or -N(CH₃)₂ subject to the proviso that at least one of is R₄ and R₅ is -NO₂, or a pharmaceutically acceptable salt or salt hydrate of such a compound, to give a compound having formula (IV) wherein R₄ and R₅ are as defined above, with the proviso that at least one of is R₄ and R₅ is -NH₂, or a pharmaceutically acceptable salt or salt hydrate of such a compound;
or
(iii) separating a compound having formula (I) wherein R₁ and R₂ are as defined under formula (I) in the form of an enantiomeric mixture so as to isolate a particular enantiomeric form;
or
(iv) converting a compound having formula (I) wherein R₁ and R₂ are as defined under formula (I) into a pharmaceutically acceptable salt or salt hydrate thereof by addition of an acid.

## Patentansprüche

1. Verbindung der Formel (I): 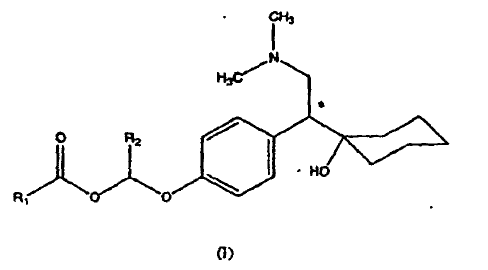 worin:
die Konfiguration am stereogenen Zentrum (*) R, S oder RS (das Racemat) sein kann;
R₁ ausgewählt wird aus C₁-C₆-Alkyl, C₁-C₆-Alkoxy, C₃-C₆-Cycloalkyl oder der Komponente: 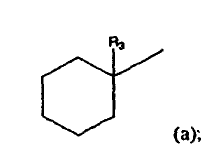
R₂ ausgewählt wird aus H oder C₁-C₆-Alkyl; oder
R₁ und R₂ verkettet werden können, so dass 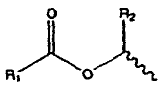 eine Komponente mit der Formel (b): 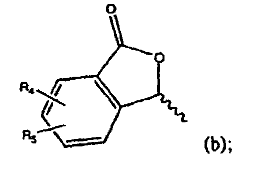 bildet;
R₃ ausgewählt wird aus H oder C₁-C₆-Alkyl; und
R₄ und R₅ unabhängig ausgewählt werden aus H, C₁-C₆-Alkyl, C₃-C₆-Cycloalkyl, C₁-C₆-Alkoxy, C₁-C₆-Thioalkoxy, -CN, -OH, -CF₃, - OCF₃, Halogen, -NH₂, -NO₂ oder Mono- oder Dialkylamino, wobei jede Alkylgruppe 1 bis 6 Kohlenstoffatome hat, oder ein pharmazeutisch annehmbares Salz oder Hydrat davon.

2. Verbindung nach Anspruch 1, worin R₁ für C₁-C₆-Alkyl oder C₁-C₆-Alkoxy steht.

3. Verbindung nach Anspruch 1 oder Anspruch 2, worin R₂ für C₁-C₆-Alkyl steht.

4. Verbindung nach Anspruch 1, worin R₁ und R₂ verkettet sind, so dass 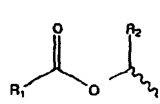 eine Komponente mit der Formel (b): 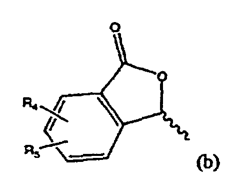 bildet
und R₄ und R₅ Wasserstoff darstellen.

5. Verbindung nach Anspruch 1, welche {4-[2-(Dimethylamino)-1-(1-hydroxy-cyclohexyl)ethyl]phenoxy}-methylpivalat ist, oder ein pharmazeutisch annehmbares Salz oder Hydrat davon.

6. Verbindung nach Anspruch 1, welche 1-{4-[2-(Dimethylamino)-1-(1-hydroxy-cyclohexyl)ethyl]phenoxy}ethylpropionat ist, oder ein pharmazeutisch annehmbares Salz oder Hydrat davon.

7. Verbindung nach Anspruch 1, welche 3-{4-[2-Dimethylamino)-1-(1-hydroxy-cyclohexyl)ethyl]phenoxy}-2-benzofuran-1(3H)-on ist, oder ein pharmazeutisch annehmbares Salz oder Hydrat davon.

8. Pharmazeutische Zusammensetzung, welche eine Verbindung der Formel I, wie in einem der Ansprüche 1 bis 7 beansprucht, oder ein pharmazeutisch annehmbares Salz oder Hydrat davon; und einen pharmazeutisch annehmbaren Träger oder Exzipienten umfasst.

9. Verwendung einer Verbindung zum Herstellen eines Medikaments zum Behandeln von Störungen des zentralen Nervensystems in einem Säuger, wobei die verwendete Verbindung eine Verbindung der Formel I ist, wie in einem der Ansprüche 1 bis 7 beansprucht, oder ein pharmazeutisch annehmbares Salz oder Hydrat davon.

10. Verwendung nach Anspruch 9, wobei die Störung des zentralen Nervensystems ausgewählt wird aus einer oder mehreren der folgenden: Depression; generalisierte Angststörung; Panikstörung; Post-traumatische Stress-Störung; Aufmerksamkeitsdefizit-Störung mit oder ohne Hyperaktivität; Angstzustände; Schizophrenie; Cocain-Abhängigkeit; Alkohol-Abhängigkeit; prämenstruelle dysphorische Störung und Autismus.

11. Verwendung nach Anspruch 9, wobei die Störung des zentralen Nervensystems Anorexia nervosa, Bulimia nervosa, vasomotorisches Erröten und chronisches Müdigkeitssyndrom ist.

12. Verwendung nach Anspruch 9, wobei die Störung des zentralen Nervensystems Harninkontinenz ist.

13. Verwendung nach Anspruch 9, wobei die Störung des zentralen Nervensystems Schmerz ist.

14. Verwendung nach Anspruch 9, wobei die Störung des zentralen Nervensystems sexuelle Dysfunktion ist.

15. Verwendung einer Verbindung zum Herstellen eines Medikaments zum Verstärken von Kognition in einem Säuger, wobei die verwendete Verbindung eine Verbindung der Formel I ist, wie in einem der Ansprüche 1 bis 7 beansprucht, oder ein pharmazeutisch annehmbares Salz oder Hydrat davon.

16. Verfahren zum Herstellen einer Verbindung mit der Formel I wie in Anspruch 1 beansprucht oder eines pharmazeutisch annehmbaren Salzes oder Hydrats davon, welches Verfahren eines der folgenden umfasst:
(i) Umsetzen von R-, S- oder (R/S)-4-[2-(Dimethylamino-1-(1-hydroxycyclohexyl)-ethyl]-phenol der Formel: 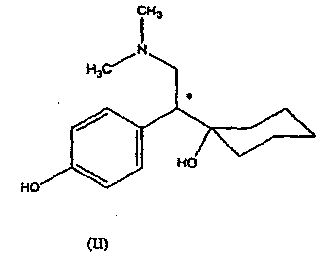 mit einer Verbindung mit der Formel (V)
R₁-CO-CHR₂-X (V),
worin R₁ und R₂ wie oben definiert sind, mit der Maßgabe, dass ein -OH oder -NH₂ Substituent an der verketteten R₁- und R₂-Gruppe durch eine Schutzgruppe geschützt werden kann, die nachfolgend entfernt wird, und X eine Abgangsgruppe darstellt; oder
(ii) Reduzieren einer Verbindung mit der Formel (IV) 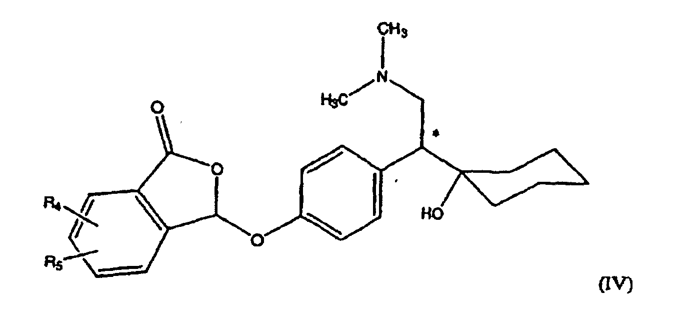 worin die Konfiguration am stereogenen Zentrum (*) R, S oder RS (das Racemat) sein kann und R₄ und R₅ unabhängig ausgewählt werden aus H, C₁-C₆-Alkyl, C₁-C₆-Alkoxy; C₁-C₆-Thioalkoxy, -CN, -OH, -CF₃, -OCF₃, Halogen, -NH₂, -NO₂ oder -N(CH₃)₂ mit der Maßgabe, dass mindestens eines von R₄ und Rs für -NO₂ steht, oder ein pharmazeutisch annehmbares Salz oder Salzhydrat einer solchen Verbindung, um eine Verbindung mit der Formel (IV) zu ergeben, worin R₄ und R₅ wie oben definiert sind, mit der Maßgabe, dass mindestens eines von R₄ und R₅ für -NH₂ steht, oder ein pharmazeutisch annehmbares Salz oder Salzhydrat einer solchen Verbindung; oder
(iii) Trennen einer Verbindung mit der Formel (I), worin R₁ und R₂ wie unter Formel (I) definiert sind, in die Form eines enantiomeren Gemisches, um so eine bestimmte enantiomere Form zu isolieren; oder
(iv) Umwandeln einer Verbindung mit der Formel (I), worin R₁ und R₂ wie unter Formel (I) definiert sind, in ein pharmazeutisch annehmbares Salz oder Salzhydrat davon durch Zugabe einer Säure.

## Revendications

1. Composé de formule I: 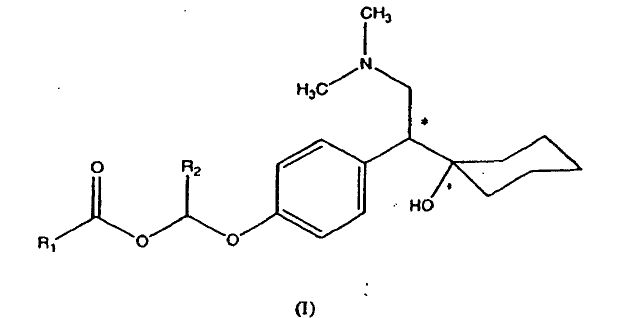 dans laquelle
la configuration au niveau du centre stéréogénique (*) peut être R, S ou RS (les racémiques); R₁ est choisi parmi un alkyle en C₁-C₆, un alkoxy en C₁-C₆, un cycloalkyle en C₃-C₆ ou le groupement: 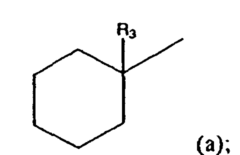 R₂ est choisi parmi H ou un alkyle en C₁-C₆, ou R₁ et R₂ peuvent être concaténés de sorte que 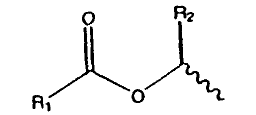 forment un groupement de formule (b): 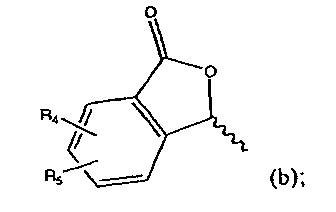 R₃ est choisi parmi H ou un alkyle en C₁-C₆ et R₄ et R₅ sont indépendamment choisis parmi H, un alkyle en C₁-C₆, un cycloalkyle en C₃-C₆, un alkoxy en C₁-C₆, un thioalkoxy en C₁-C₆, -CN, -OH, -CF₃, -OCF₃, un halogène, -NH₂, -NO₂ ou un mono- ou dialkylamino, où chaque groupe alkyle a 1 à 6 atomes de carbone ou un sel ou un hydrate pharmaceutiquement acceptable de ceux-ci.

2. Composé selon la revendication 1, dans lequel R₁ est un alkyle en C₁-C₆ ou un alkoxy en C₁-C₆.

3. Composé selon la revendication 1 ou la revendication 2, dans lequel R₂ est un alkyle en C₁-C₆.

4. Composé selon la revendication 1, dans lequel R₁ et R₂ sont concaténés de sorte que 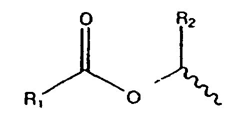 forment un groupement de formule (b); 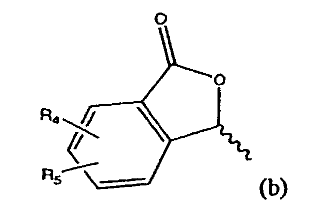 et R₄ et R₅ sont un hydrogène.

5. Composé selon la revendication 1 qui est un pivalate de {4-[2-(diméthylamino)-1-(1-hydroxycyclohexyl)éthyl]phénoxy}méthyle ou un sel ou un hydrate pharmaceutiquement acceptable de celui-ci.

6. Composé selon la revendication 1 qui est un propionate de 1-{4-[2-(diméthylamino)-1-(1-hydroxycyclohexyl)éthyl]phénoxy}éthyle ou un sel ou un hydrate pharmaceutiquement acceptable de celui-ci.

7. Composé selon la revendication 1 qui est une 3-{4-[2-(diméthylamino)-1-(1-hydroxycyclohexyl)éthyl]phénoxy}-2-benzofuranne-1-(3H)-one ou un sel ou un hydrate pharmaceutiquement acceptable de celle-ci.

8. Composition pharmaceutique comprenant un composé de formule I selon l'une quelconque des revendications 1 à 7 ou un sel ou un hydrate pharmaceutiquement acceptable de celui-ci; et un véhicule ou un excipient pharmaceutiquement acceptable.

9. Utilisation d'un composé pour la préparation d'un médicament destiné à traiter les troubles du système nerveux central chez un mammifère, dans laquelle le composé utilisé est un composé de formule I selon l'une quelconque des revendications 1 à 7 ou un sel ou un hydrate pharmaceutiquement acceptable de celui-ci.

10. Utilisation selon la revendication 9, dans laquelle le trouble du système nerveux central est choisi parmi un ou plusieurs des troubles suivants: la dépression, un trouble de l'anxiété généralisé, la panique, un stress post-traumatique, un trouble du déficit d'attention avec ou sans hyperactivité ; de l'anxiété, la schizophrénie, la cocaïnomanie, l'alcoolisme, un trouble dysphorique prémenstruel et l'autisme.

11. Utilisation selon la revendication 9, dans laquelle le trouble du système nerveux central est l'anorexie nerveuse, la boulimie nerveuse, une bouffée vasomotrice et le syndrome de fatigue chronique.

12. Utilisation selon la revendication 9, dans laquelle le trouble du système nerveux central est une incontinence urinaire.

13. Utilisation selon la revendication 9, dans laquelle le trouble du système nerveux central est une douleur.

14. Utilisation selon la revendication 9, dans laquelle le trouble du système nerveux central est un dysfonctionnement sexuel.

15. Utilisation d'un composé pour la fabrication d'un médicament destiné à favoriser la cognition chez un mammifère, dans laquelle le composé utilisé est un composé de formule I selon l'une quelconque des revendications 1 à 7 ou un sel ou un hydrate pharmaceutiquement acceptable de celui-ci.

16. Procédé pour la préparation d'un composé ayant la formule I selon la revendication I, ou d'un sel
ou un hydrate pharmaceutiquement acceptable de celui-ci, lequel procédé comprend une des étapes suivantes:
(i) la réaction de R-, S- ou (R/S)-4[2-(diméthylamino-1-(1-hydroxycyclohexyl)éthyl]phénol de formule: 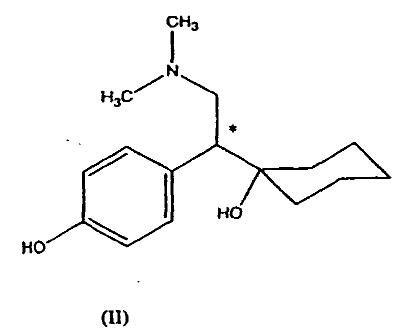 avec un composé ayant la formule (V)
R₁-CO-CHR₂-X (V)
dans laquelle R₁ et R₂ sont tels que définis précédemment à condition que un substituant -OH ou un substituant -NH₂ sur le groupe R₁ et R₂ concaténé puisse être protégé par un groupe protecteur qui est ensuite supprimé et X est un groupe partant; ou
(ii) la réduction d'un composé ayant la formule (IV) 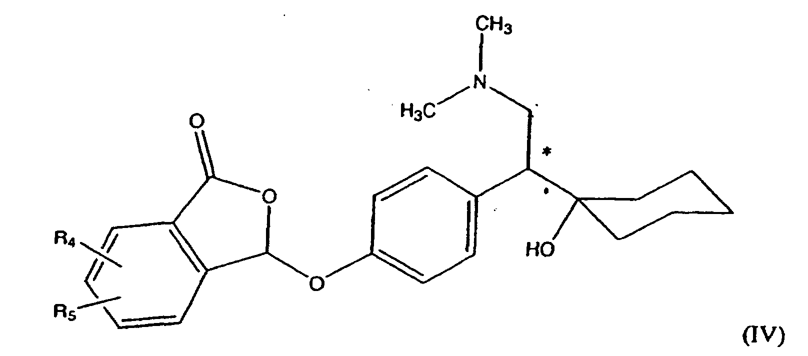 dans laquelle la configuration au niveau du centre stéréogénique (*) peut être R, S ou RS (les racémiques) et R₄ et R₅ sont indépendamment choisis parmi H, un alkyle en C₁-C₆, un alkoxy en C₁-C₆, un thioalkoxy en C₁-C₆, -CN, -OH, -CF₃-OCF₃, un halogène -NH₂, -NO₂ ou -N(CH₃)₂, à condition qu'au moins un soit R₄ et R₅ est -NO₂ ou un sel ou un hydrate de sel pharmaceutiquement acceptable d'un tel composé pour donner un composé ayant la formule (IV) dans laquelle R₄ et R₅ sont tels que définis précédemment à condition qu'au moins un de R₄ et R₅ soit NH₂ ou un sel ou un hydrate de sel pharmaceutiquement acceptable d'un tel composé;
ou
(iii) la séparation d'un composé ayant la formule (I) dans laquelle R₁ et R₂ sont tels que définis pour la formule (I) sous la forme d'un mélange énantiomère de façon à isoler une forme particulière d'énantiomère;
ou
(iv) la conversion d'un composé ayant la formule (I) dans laquelle R₁ et R₂ sont tels que définis pour la formule (I) en un sel ou un hydrate de sel pharmaceutiquement acceptable de celui-ci par addition d'un acide.
